# DEMANDE DE BREVET EUROPEEN

(11) **EP 1 036 555 A1**
(43) Date de publication de la demande: **20.09.2000**
(21) Numéro de dépôt: 99403052.6
(22) Date de dépôt: 07.12.1999
(51) Int. Cl.: A61K 7/035, A61K 7/48

(54) **Composition cosmétique hydrophobe anhydre sous forme de poudre compacte**

(30) Priorité: 30.12.1998 FR 9816662
(71) Demandeur: L'OREAL, 75008 Paris (FR)
(72) Inventeur: Cantin, Hervé, 91420 Morangis (FR)
(74) Mandataire: Brédeville, Odile Marie

(57) **Abrégé**

L'invention se rapporte à une composition cosmétique hydrophobe anhydre sous forme de poudre compacte pouvant être prise à sec ou à l'eau, comprenant une phase particulaire comprenant des composés pulvérulents hydrophiles, caractérisée en ce que les composés pulvérulents hydrophiles représentent 20 à 35 % en poids du poids total de la phase particulaire. Elle se rapporte également à un procédé de maquillage mettant en oeuvre une telle composition ainsi qu'à un dispositif comprenant une telle composition et une éponge susceptible d'être humidifiée.

## Description

La présente invention concerne des "two-way cakes" ou encore des compositions cosmétiques sous forme de poudres qui peuvent être utilisées à sec, telles quelles, ou à l'eau grâce à une éponge humide.

Les "two-way cakes" sont des poudres cosmétiques particulières : elles sont sous la forme de produits compactés et présentent l'avantage de pouvoir être prises soit à l'éponge sèche ou applicateur sec, et elles sont alors utilisées comme des poudres classiques, soit à l'eau à l'aide d'une éponge humide, et elles peuvent être alors utilisées comme des fonds de teint.

De tels produits sont particulièrement appréciés des consommatrices : en effet, ils sont pratiques, compacts, faciles à utiliser, permettent une double utilisation à la fois comme poudre et comme fond de teint tout en ne prenant la place que d'un seul produit. Appliqués à sec, ils procurent une sensation de douceur et de non-gras. Appliqués à l'eau, ils procurent une sensation de fraîcheur.

La composition des "two-way cake" est celle des poudres classiques. Les "two-way cakes" comprennent ainsi généralement entre 75 et 99 % de composés pulvérulents et entre 1 à 25 % de liant, c'est-à-dire de composés huileux permettant la cohésion des composés pulvérulents.

Toutefois, dans la mesure où les "two-way cakes" doivent pouvoir être pris avec une éponge humide, ils doivent être hydrophobes. En effet, dans le cas contraire, l'eau de l'éponge humide serait incorporée dans le "two-way cake", l'éponge collerait au produit et il serait impossible de déliter une partie du produit afin de l'appliquer ensuite sur le visage par exemple. Par produit hydrophobe, on entend ici un produit qui n'incorpore pas une goutte d'eau que l'on dépose sur sa surface : la goutte d'eau roule sur ladite surface.

La plupart des "two-way cakes" connus sont des produits qui ne comprennent que des charges hydrophobes et des huiles : ils sont ainsi complètement hydrophobes.

Mais les composés pulvérulents employés dans les poudres ne sont pas tous naturellement hydrophobes. On incorpore donc de façon classique, dans les "two-way cakes", des composés naturellement hydrophobes et des composés qu'on a rendus hydrophobes par un enrobage particulier, le plus souvent avec une silicone, un aminoacide ou encore du polyéthylène. On obtient ainsi un produit qui ne contient que des composés pulvérulents hydrophobes.

L'inconvénient de ces produits est qu'ils sont chers et difficiles à fabriquer. En effet, l'enrobage d'un composé pulvérulent est une opération longue et coûteuse.

Or la Demanderesse a trouvé, de façon surprenante, qu'il était possible de réaliser des "two-way cakes" économiques et faciles à fabriquer en choisissant un intervalle particulier de composés pulvérulents hydrophiles, ces "two-way cakes" conservant par ailleurs d'excellentes propriétés cosmétiques et de tenue dans le temps.

Cette découverte est à l'origine de l'invention.

La présente invention porte donc sur un "two-way cake", ou encore composition cosmétique hydrophobe anhydre sous forme de poudre compacte pouvant indifféremment être prise à sec ou à l'eau, comprenant une phase particulaire comprenant des composés pulvérulents hydrophiles, caractérisée en ce que les composés pulvérulents hydrophiles sont présents à une teneur allant de 20 à 35 % en poids, par rapport au poids total de la phase particulaire.

La composition selon l'invention est de réalisation extrêmement simple et peut être fabriquée selon les méthodes classiques de préparation des poudres cosmétiques.

Comme elle comprend peu de poudres rendues hydrophobes par un traitement, voire pas du tout, elle n'est pas coûteuse.

Elle possède d'excellentes propriétés cosmétiques, se délite facilement aussi bien à sec qu'à l'eau et s'étale de façon remarquable sur la peau. La composition selon l'invention présente une texture extrêmement fine au toucher : de plus, une fois maquillée, la composition est toujours aussi fine. Elle semble conférer ainsi à la peau une texture naturellement fine et douce.

La composition selon l'invention résiste à la sueur et au sébum. Elle présente un très bonne tenue sans rendre la peau collante ou brillante, en particulier sur le nez ou le front (T-zone). La composition selon l'invention rend le teint uniforme.

La présente invention porte encore sur un procédé cosmétique de maquillage ou de soin de la peau, en particulier du corps, ou des muqueuses (lèvres, intérieurs des paupières inférieures) des êtres humains, comprenant l'application sur la peau, le corps ou les muqueuses de la composition telle que définie ci-dessus.

D'autres caractéristiques, aspects et avantages de l'invention apparaîtront à la lecture de la description détaillée qui va suivre.

Les compositions de l'invention sont des poudres cosmétiques : elles peuvent donc comprendre jusqu'à 100% en poids, de préférence de 77 à 99% en poids, par rapport au poids total de la composition, de phase particulaire.

La phase particulaire comprend des composés pulvérulents habituellement utilisés dans la fabrication des poudres cosmétiques.

Les composés pulvérulents peuvent être choisis parmi les charges, les pigments et/ou les nacres, et/ou leurs mélanges.

Par charges, il faut comprendre des particules incolores ou blanches, minérales ou organiques, lamellaires ou non lamellaires, destinées à donner du corps ou de la rigidité à la composition, et/ou de la douceur, de la matité et de l'uniformité au maquillage. Par pigments, il faut comprendre des particules blanches ou colorées, minérales ou organiques, destinées à colorer et/ou opacifier la composition. Par nacres, il faut comprendre des particules irisées qui réfléchissent la lumière.

Les charges peuvent être présentes dans la composition de l'invention à une teneur pouvant aller de 0,1 à 99 % en poids, par rapport au poids total de la composition.

Parmi ces charges, on peut citer par exemple les silicates minéraux comme le mica. Le mica peut être choisi parmi la muscovite, la phlogopite, la tiotite, la séricite, la lépidolite, la paragonite, les mica synthétiques et leurs mélanges. De préférence, on utilise la séricite naturelle ou de synthèse, par exemple à une teneur allant de 1 à 30% en poids, par rapport au poids total de la composition.

Comme autre charge, on peut également citer le talc qui peut par exemple être utilisé à une teneur pouvant aller 1 à 85% en poids, par rapport au poids total de la composition.

Comme autres charges, on peut également citer la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses telles que l'Expancel (Nobel Industrie), les microéponges comme le polytrap (Dow Corning) et les microbilles de résine de silicone (Tospearls de la Société TOSHIBA, par exemple), les oxydes de zinc et de titane, les oxydes de zirconium ou de cérium, le carbonate de calcium précipité, le carbonate et l'hydrocarbonate de magnésium, l'hydroxyapatite, les microsphères de silice creuses (Silica Beads de la Société MAPRECOS), les microcapsules de verre ou de céramique ; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et/ou leurs mélanges.

Les charges peuvent également se présenter sous la forme de composés à structure composite, comme par exemple des charges composées de plusieurs couches de matériaux pulvérulents différents : on peut citer les composés SiO₂/TiO₂/ SiO₂, TiO₂/CeO₂/SiO₂, ou encore TiO₂/ZnO/Talc.

Les pigments peuvent être présents dans la composition de l'invention à une teneur pouvant aller de 0,01% à 30%, de préférence de 0,1% à 5%, en poids par rapport au poids total de la composition.

Ils peuvent être de taille usuelle ou nanométrique.

On peut citer, parmi les pigments et les nanopigments minéraux, les dioxydes de titane, les oxydes de zinc, de fer ou de chrome, les nanotitanes, le bleu ferrique.

Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques, les pigments de type D&C, les laques à base de carmin de cochenille, et/ou leurs mélanges.

Les nacres, qui peuvent être présentes à raison de 0-50 % en poids, peuvent être choisies parmi les pigments nacrés tels que le mica recouvert de pigments organiques et/ou minéraux tel que le dioxyde de titane ou l'oxychlorure de bismuth, le mica titane recouvert de pigments organiques et/ou minéraux tel que les oxydes de fer, le bleu ferrique ou l'oxyde de chrome, ainsi que les pigments nacrés à base d'oxychlorure de bismuth.

Ces composés pulvérulents sont naturellement hydrophobes ou hydrophiles.

Pour déterminer si, selon l'invention, une matière pulvérulente est "hydrophobe" ou "hydrophile", on effectue le test ci-après défini. On remplit avec 20 ml d'eau un tube à essai d'un diamètre de 20 mm. On verse 2 grammes de poudre dans le tube sans agiter et on observe le comportement de la poudre pendant 5 minutes au maximum. Si la poudre reste parfaitement en surface, elle est considérée comme "hydrophobe". Dans le cas contraire, elle est considérée comme "hydrophile".

Les composés pulvérulents hydrophiles sont présents dans les compositions selon l'invention à une teneur allant de 20 à 35 % en poids, par rapport au poids total de la phase particulaire. La part restante de la phase particulaire est constituée de composés pulvérulents hydrophobes.

Les composés pulvérulents hydrophiles peuvent être hydrophiles naturellement ou peuvent avoir été rendus hydrophiles.

Les composés hydrophiles naturellement peuvent par exemple être choisis parmi:
- les micas, qui sont des silicates d'aluminium et de potassium de compositions variées, d'origine naturelle, tels que la muscovite, la phlogopite, la lépidolite, la biotite et la séricite, ou d'origine synthétique,
- l'oxychlorure de bismuth,
- les silices, qui peuvent être sous forme de plaquettes ou de sphères telles que la silice commercialisée sous la dénomination "SILICA BEADS SB 700" par la Société MIYOSHI,
- les poudres de polymères hydrophiles, qui sont d'origine synthétique comme les polyacrylates, par exemple celui commercialisé sous la dénomination "MICROPEARL M 100" par la Société MATSUMOTO, les polyamides acryliques tels que ceux commercialisés par la Société ORIS, les polyuréthannes tels que celui commercialisé sous la dénomination "PLASTIC POWDER D 800" par la Société TOSHNU, les dérivés de cellulose ou d'amidon, par exemple les microsphères poreuses de cellulose,
- le kaolin, qui est un silicate d'aluminium hydraté,
- l'hydroxyapatite,
- les oxydes de zinc ou de titane, pour leur couvrance notamment, ces produits pouvant être utilisés à l'état nanopigmentaire pour leur effet filtrant,
- le carbonate de calcium,
- les carbonates et hydrocarbonates de magnésium, qui facilitent la fixation des parfums,
- et/ou leurs mélanges.

Les composés pulvérulents hydrophiles peuvent également être des composés pulvérulents qui ont été rendus hydrophiles par enrobage ou greffage chimique à l'aide de matières telles que le chitosane, le dioxyde de titane, la silice ou des polymères hydrophiles, notamment des polyesters sulfoniques ou des polyammonium quaternaire, et/ou leurs mélanges.

Ainsi, les composés pulvérulents hydrophiles peuvent être choisis parmi des talcs traités et/ou enrobés hydrophiles, des poudres de polyamide traitées et/ou enrobées hydrophiles, des poudres de polyéthylène traitées et/ou enrobées hydrophiles, des poudres de copolymère expansé de chlorure de vinylidène, d'acrylonitrile et de (méth)acrylate de méthyle traitées et/ou enrobées hydrophiles, des poudres polyfluorées traitées et/ou enrobées hydrophiles, des poudres de silicone traitées et/ou enrobées hydrophiles, des poudres de copolymère acrylique traitées et/ou enrobées hydrophiles, des poudres de polystyrène traitées et/ou enrobées hydrophiles, des pigments traités et/ou enrobés hydrophiles, et/ou leurs mélanges.

A titre d'exemples de composés pulvérulents hydrophiles, on peut citer le talc enrobé de chitosane vendu par Daito sous le nom "Talc CT 2 MSA", le mica enrobé de microsphères de silice vendu par Catalysts et Chemicals sous le nom "Cashmir B3".

Les composés pulvérulents hydrophiles peuvent également être choisis parmi les pigments hydrophiles utilisables en cosmétique.

Parmi les pigments minéraux, on peut citer, à titre d'exemple :
- les oxydes de fer noir, jaune, rouge et brun, codifiés dans le Color Index sous les références Cl 77499, CI 77492 et Cl 77491,
- le violet de manganèse (Cl 77742),
- le bleu d'outremer (Cl 77007),
- le violet d'outremer (Cl 77007),
- l'oxyde de chrome (Cl 77288),
- l'oxyde de chrome hydraté (Cl 77289) et
- le bleu ferrique (Cl 77510),
- et/ou leurs mélanges.

Parmi les pigments organiques, on peut citer, en particulier, les pigments :
- D & C red n° 3 (Cl 45430 : 1),
- D & C red n° 6 (Cl 15850 : 2),
- D & C red n° 7 (Cl 15850 : 1),
- D & C red n° 9 (Cl 15585 : 1),
- D & C red n° 13 (Cl 15630 : 3),
- D & C red n° 19 (Cl 45170),
- D & C red n° 21 (Cl 45380 : 2),
- D & C red n° 27 (Cl 45410: 1),
- D & C red n° 30 (Cl 73360),
- D & C red n° 36 (Cl 12085),
- le noir de carbone (Cl 77266) et les laques à base de carmin de cochenille (Cl 75470),
- et/ou leurs mélanges.

Les composés pulvérulents hydrophobes peuvent être choisis parmi les composés pulvérulents hydrophobes par nature comme par exemple :
- le talc qui est un silicate de magnésium hydraté,
- les poudres de polymères hydrophobes, telles que la poudre des polyamides de type nylon, par exemple celle commercialisée sous la dénomination "ORGASOL 2002 ED NAT COS" par la Société ATOCHEM, la poudre de polyéthylène, par exemple celle commercialisée sous la dénomination "COATHYLENE HA 1681" par la Société PLAST LABOR ; les microsphères expansées en matériau thermoplastique, par exemple celle commercialisée sous la dénomination "EXPANCEL 551 DE" par la Société CASCO-NOBEL, les poudres polyfluorées notamment de polytétrafluoroéthylène, par exemple celle commercialisée sous la dénomination "MP 1400" par la Société DUPONT DE NEMOURS, les poudres de silicone, par exemple celles commercialisées sous la dénomination "TOSPEARL" par la Société TOSHIBA, les poudres de copolymère acrylique, telles que celles commercialisées sous la dénomination "POLYTRAP Q5 6603" par la Société DOW CHEMICA, ou encore les poudres de polystyrène telles que celles commercialisées sous la dénomination "POLYSPHERE 3 000 SP" par la Société PRESPERESE ;
- les lipoaminoacides, par exemple la lauroyl lysine,
- le nitrure de bore,
- les savons métalliques d'acides carboxyliques en C₈-C₂₂, plus particulièrement en C₁₂-C₁₈, par exemple les stéarates de zinc et de magnésium, le laurate de zinc ou le myristate de magnésium,
- et/ou leurs mélanges.

Les composés pulvérulents hydrophobes peuvent aussi être choisis parmi des composés pulvérulents de nature aussi bien hydrophobe qu'hydrophile que l'on a rendus hydrophobes en les traitant par enrobage ou greffage chimique par des produits tels que les silicones, les aminoacides, les savons métalliques, les dérivés fluorés, les huiles minérales, la lécithine, le triisostéaroyl titanate d'isopropyle, le polyéthylène, le collagène et ses dérivés, les polyacrylates, et/ou leurs mélanges.

Ainsi, les composés pulvérulents hydrophobes peuvent être choisis parmi des micas enrobés et/ou traités hydrophobes, des silices enrobées et/ou traitées hydrophobes, du kaolin enrobé et/ou traité hydrophobe, des oxydes métalliques enrobés et/ou traités hydrophobes comme les oxydes de titane enrobés et/ou traités hydrophobes, les oxydes de fer enrobés et/ou traités hydrophobes, les oxydes de zinc enrobés et/ou traités hydrophobes, et/ou leurs mélanges.

A titre d'exemples de composés pulvérulents hydrophobes, on peut citer les microbilles de silice enrobées de polymethylhydrogenosiloxane vendues sous la dénomination commerciale "Silice SI SB 700" par Miyoshi ou encore la sericite enrobée de methicone/huile d'oeuf hydrogénée vendue sous la dénomination commerciale "Sericite SNI S100" par Miyoshi.

Les compositions selon l'invention peuvent également comprendre une phase grasse à titre de liant comprenant des corps gras et qui facilite l'adhésion sur la peau des composés pulvérulents ainsi que leur cohésion entre eux au sein de la composition finale.

Ces corps gras peuvent être choisis parmi les huiles et/ou cires d'origine minérale, animale ou végétale, les huiles de silicone, les huiles fluorées, les esters d'acide gras, et/ou leurs mélanges.

Parmi les huiles utilisables, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, et/ou leurs mélanges.

De préférence, on choisira les huiles de silicone telles que les polyméthylsiloxanes, les polyméthylphénylsiloxanes, et/ou leurs mélanges.

Parmi les cires utilisables, on peut citer les cires d'abeille, les cires de lanoline et les cires d'insectes de Chine ; les cires de Carnauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères cireux ainsi que leurs esters, et les cires de silicone telles que les polyalcoxy et polyalkylsiloxanes, et/ou leurs mélanges.

La phase grasse peut également comprendre des résines de silicone.

La phase grasse est de préférence présente dans les compositions selon l'invention à une teneur pouvant aller de 0,1 à 25 % en poids, de préférence de 3 à 15%, par rapport au poids total de la composition.

La phase grasse peut, en outre, comprendre des additifs tels que des actifs cosmétiques lipophiles et/ou des ingrédients liposolubles généralement utilisés en cosmétique comme les parfums, les filtres solaires. De préférence, ces additifs peuvent être présents en une proportion allant de 20 à 70% en poids, par rapport au poids total de la phase grasse.

La composition de l'invention peut comprendre, en outre, tout additif usuellement utilisé dans le domaine concerné des antioxydants, des huiles essentielles, des conservateurs, des neutralisants, des tensio-actifs E/H ou H/E, des vitamines, des actifs antirides, et/ou leurs mélanges.

Les compositions selon l'invention sont sous forme de poudre compacte ou encore pressée. Elles sont hydrophobes et anhydres. Par anhydre, on entend une composition substantiellement exempte d'eau, de préférence, une composition dans laquelle l'eau est présente à une teneur inférieure ou égale à 2% en poids, par rapport au poids total de la composition.

Les compositions selon l'invention sont préparées selon les méthodes classiques de préparation des poudres compactes ou pressées. Les composés pulvérulents sont mélangés. La phase grasse est ensuite ajoutée au goutte à goutte et on mélange à nouveau. Le mélange obtenu est ensuite broyé puis tamisé pour desagglomérer le mélange. La poudre est ensuite compactée dans une coupelle.

Les compositions selon l'invention peuvent s'utiliser indifféremment à sec ou à l'eau. Lorsqu'elles sont utilisées à sec, elles peuvent être prélevées avec le doigt ou avec une éponge ou une houppette sèche : elles sont alors utilisées comme des poudres, par exemple par-dessus une crème hydratante ou un fond de teint. Elles procurent alors une sensation de douceur, de non-gras. Elles confèrent à la peau un aspect velouté, aérien.

Ces compositions peuvent également être utilisées à l'eau grâce à un applicateur que l'on a préalablement humidifié et à l'aide duquel on délite une partie de la poudre. Celle-ci se mélange à l'eau et la composition est alors utilisée sur la peau comme un fond de teint. Elle procure ainsi une sensation de fraîcheur et confère à la peau un effet matifiant, couvrant, unifiant du teint.

Le même applicateur peut convenir pour les deux utilisations des compositions de l'invention. Cet applicateur doit être suscetible d'être humidifié. Il peut par exemple être une éponge ou une houppette constituée d'un ou de plusieurs matériaux d'origine naturelle ou synthétique comme par exemple les mousses de polyuréthane, de polyester, de polyéther, de polychlorure de vinyle, de polyéthylène. Il est choisi pour ses qualités cosmétiques comme la douceur, l'épaisseur. Il n'est ni trop rigide ni trop mou. De préférence, l'applicateur est une éponge en polymère de type NBR densité medium.

La présente invention a encore pour objet un dispositif comprenant une composition telle que décrite ci-dessus et un applicateur susceptible d'être humidifié, de préférence une éponge.

L'invention est illustrée plus en détail dans les exemples suivants. Les pourcentages sont donnés en poids par rapport au poids total de la composition.

### Exemple 1 :

La Demanderesse a réalisé le two-way cake conforme à l'invention suivant :

### phase A

- Talc hydrophobe 47,1 %

### phase B

- Oxyde de fer hydrophile 0,8 %
- Oxyde de fer hydrophile 0,8 %
- Oxyde de fer hydrophile 0,3 %
- Mica hydrophile 20 %
- Dioxyde de titane hydrophile 5 %
- Nylon hydrophobe 10 %
- Laurate de zinc hydrophobe 5 %
- Lauroyl lysine hydrophobe 2,7 %
- Paraben hydrophile 0,3 %

### phase C

Liant hydrophobe 8 %

Cette composition a été réalisée par mélange de la phase A et de la phase B puis ajout de la phase C. Le mélange obtenu a ensuite été broyé, tamisé puis compacté.

Cette composition comprend 29,5% en poids, par rapport au poids de la phase particulaire, de composés pulvérulents hydrophiles. Elle est hydrophobe. Elle peut s'utiliser à sec ou à l'eau. Elle comprend peu de charges enrobées et est par conséquent très peu coûteuse et très simple à fabriquer.

Elle se délite facilement à l'éponge humide comme à l'éponge sèche.

### Exemple 2 : comparatif

La Demanderesse a réalisé la composition suivante :

### Phase A :

- talc hydrophobe 36.6%

### Phase B :

- pigments hydrophiles 1.6%
- poudre de nylon hydrophobe 20%
- magnésie hydrophile 1,8%
- oxychlorure de bismuth hydrophile 10%
- stéarate de zinc hydrophobe 4%
- mica hydrophile 20%

### Phase C :

- liant hydrophobe 6%

Cette composition a été réalisée de la même façon que la composition de l'exemple 1.

Elle comprend 35,5% en poids, par rapport au poids de la phase particulaire, de composés pulvérulents hydrophiles. Lorsqu'on tente de prélever une partie de la poudre à l'aide d'une éponge humide ou mouillée, il se forme des agglomérats de poudre inutilisable sur l'éponge. Il n'est pas possible de déliter une telle composition pour une application cosmétique sur le visage.

## Revendications

1. Composition cosmétique hydrophobe anhydre sous forme de poudre compacte comprenant une phase particulaire comprenant des composés pulvérulents hydrophiles, caractérisée en ce que les composés pulvérulents hydrophiles sont présents à une teneur allant de 20 à 35 % en poids, par rapport au poids total de la phase particulaire.

2. Composition selon la revendication 1, caractérisée par le fait qu'elle est substantiellement exempte d'eau.

3. Composition selon la revendication 2, caractérisée par le fait que l'eau est présente à une teneur inférieure ou égale à 2% en poids, par rapport au poids total de la composition.

4. Composition selon la revendication 1, caractérisée par le fait que la phase particulaire est présente à une teneur pouvant aller de 77 à 99%, en poids, par rapport au poids total de la composition.

5. Composition selon l'une quelconque des revendications précédentes, caractérisée par le fait que la phase particulaire comprend des composés pulvérulents choisis parmi les charges, les pigments et/ou les nacres, et/ou leurs mélanges.

6. Composition selon la revendication précédente, caractérisée par le fait que les charges sont choisies parmi le mica, le talc, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses, les microéponges et les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et/ou leurs mélanges.

7. Composition selon la revendication précédente, caractérisée par le fait qu'elle comprend de 1 à 85% en poids, par rapport au poids total de la composition, de talc.

8. Composition selon l'une quelconque des revendications 5 à 7, caractérisée par le fait que les pigments sont présents à une teneur allant de 0,01% à 30%, de préférence de 0,1% à 5%, en poids par rapport au poids total de la composition.

9. Composition selon la revendication précédente, caractérisée par le fait que les pigments sont choisis parmi les dioxydes de titane, les oxydes de zinc, de fer ou de chrome, les nanotitanes, le bleu ferrique, le noir de carbone, les sels de calcium, de baryum, d'aluminium ou de zirconium, de colorants acides tels que les colorants halogéno-acides, azoïques ou anthraquinoniques, les pigments de type D&C, les laques à base de carmin de cochenille, et/ou leurs mélanges.

10. Composition selon l'une quelconque des revendications précédentes, caractérisée par le fait que les composés pulvérulents hydrophiles sont choisis parmi les micas d'origine naturelle, tels que la muscovite, la phlogopite, la lépidolite, la biotite et la séricite, ou d'origine synthétique, l'oxychlorure de bismuth, les silices, les poudres de polymères hydrophiles comme les polyacrylates, les polyamides acryliques, les polyuréthannes, les dérivés de cellulose ou d'amidon, le kaolin, l'hydroxyapatite, les oxydes de zinc ou de titane, le carbonate de calcium, les carbonates et hydrocarbonates de magnésium, les composés pulvérulents rendus hydrophiles par enrobage ou greffage chimique à l'aide de matières telles que le chitosane, le dioxyde de titane, la silice ou des polymères hydrophiles, notamment des polyesters sulfoniques ou des polyammonium quaternaire, les pigments hydrophiles, et/ou leurs mélanges.

11. Composition selon la revendication précédente, caractérisée par le fait qu'elle comprend en outre une phase grasse.

12. Composition selon la revendication précédente, caractérisée par le fait que la phase grasse comprend des huiles choisies parmi les polyméthylsiloxanes et les polyméthylphénylsiloxanes, et/ou leurs mélanges.

13. Procédé cosmétique de maquillage ou de soin de la peau, en particulier du corps, ou des muqueuses des êtres humains, comprenant l'application sur la peau, le corps ou les muqueuses d'une composition telle que définie à l'une quelconque des revendications 1 à 12.

14. Dispositif comprenant une composition telle que définie à l'une quelconque des revendications 1 à 12 et un applicateur susceptible d'être humidifié, de préférence une éponge.
